# EUROPÄISCHE PATENTSCHRIFT

(11) **EP 3 177 232 B1**
(45) Veröffentlichungstag und Bekanntmachung des Hinweises auf die Patenterteilung: **04.09.2019**
(21) Anmeldenummer: 15747353.9
(22) Anmeldetag: 15.07.2015
(51) Int. Cl.: A61F 2/28, A61F 2/30

(54) **VERFAHREN ZUR HERSTELLUNG EINER MEHRSCHICHTIGEN FOLIE**
METHOD FOR PRODUCING A MULTILAYER FILM
PROCÉDÉ DE PRODUCTION D'UN FILM MULTICOUCHE

(30) Priorität: 05.08.2014 AT 6222014
(43) Veröffentlichungstag der Anmeldung: 14.06.2017
(73) Patentinhaber: Sonnleitner, Dietmar, 5020 Salzburg (AT)
(72) Erfinder: Sonnleitner, Dietmar, 5020 Salzburg (AT)
(74) Vertreter: Torggler & Hofinger Patentanwälte
(86) Internationale Anmeldenummer: PCT/AT2015/000098
(87) Internationale Veröffentlichungsnummer: WO 2016/019404

(56) Entgegenhaltungen:
- WO-A1-00/15152
- WO-A1-92/10218
- WO-A1-2012/075004
- WO-A1-2014/086913
- WO-A2-2008/021921
- DE-C1- 4 313 192
- US-A- 5 380 328
- US-A1- 2013 288 199

## Beschreibung

Die Erfindung betrifft ein Verfahren zur Herstellung einer mehrschichtigen Folie gemäß dem Oberbegriff des Anspruchs 1 sowie eine mehrschichtige Folie zur Abdeckung einer Knochendefektstelle gemäß dem Oberbegriff des Anspruchs 5.

Bekannte Folien oder Membranen zur Abdeckung von Knochendefektstellen (siehe z.B. WO 00/15152 A1, WO 92/10218 A1, DE 43 13 192 C1, US 5,380,328 A) werden beispielsweise im Bereich von Kieferknochen zur Kieferaugmentation eingesetzt, um einen Kieferknochen im Fall von Knochenschwund oder Knochenverlust, der bei der Extraktion eines Zahnes oder als Folge eines entzündlichen Prozesses um einen natürlichen Zahn oder ein Implantat auftreten kann, wiederaufzubauen. Derartige Folien weisen häufig eine Formstruktur aus Titan auf, die auf einer Teflonmembran angeordnet ist und die über die Knochendefektstelle angeformt wird, sodass zwischen Folie und Knochendefektstelle ein Hohlraum entsteht, in dem Knochenmaterial und bei natürlichen Zähnen auch der Zahnhalteapparat nachwachsen kann. Die Befestigung der Folie erfolgt üblicherweise mit bioresorbierbaren oder metallenen Pins oder Schrauben, die durch die Folie hindurch am Kieferknochen befestigt werden. Alternativ kann die Folie auch mit der Unterlage bzw. mit dem Kieferknochen verklebt werden. Bei gleichzeitig eingebrachten Implantaten kann die Folie auch am Implantatkopf befestigt werden. Da die Knochenregeneration mehrere Monate in Anspruch nimmt, ist nach erfolgtem Knochenaufbau mit einer Teflonmembran eine zweite Operation nötig, um die Folie bzw. Teflonmembran wieder aus dem Körper zu entfernen.

Es sind auch bereits Deckschichten aus bioresorbierbaren Materialen bekannt, die aufgrund Ihrer Bioresorbierbarkeit vom Körper wieder aufgelöst werden, beispielsweise mittels Hydrolyse. Solche Deckschichten weisen jedoch zumeist eine zu geringe Formstabilität auf, um eine die Knochenheilung fördernde Struktur während der Dauer der Knochenheilung zu ermöglichen und aufrecht zu erhalten. Der Einsatz einer solchen Deckschicht in Verbindung mit einer formgebenden Teflonmembran erfordert wiederum eine zweite Operation, um die Teflonmembran wieder aus dem Körper zu entfernen.

Aufgabe der Erfindung ist es, die vorbeschriebenen Nachteile zu vermeiden und ein gegenüber dem Stand der Technik verbessertes Verfahren zur Herstellung einer mehrschichtigen Folie sowie eine gegenüber dem Stand der Technik verbesserte mehrschichtige Folie anzugeben. Insbesondere soll eine weitere Operation zum Entfernen der Folie vermieden werden.

Diese Aufgabe wird beim erfindungsgemäßen Verfahren durch die Merkmale des Anspruchs 1 gelöst. Vorteilhafte Ausgestaltungen der Erfindung sind in den abhängigen Ansprüchen angegeben.

Es ist vorgesehen, dass die wenigstens eine Deckschicht an eine thermisch verformbare und im Wesentlichen vollständig bioresorbierbare Formschicht angelegt wird, wobei die wenigstens eine Deckschicht mit der Formschicht thermisch und/oder mechanisch verbunden, vorzugsweise verpresst, wird.

Unter thermischer Verbindung wird unter anderem verstanden, dass die Formschicht und/oder die wenigstens eine Deckschicht durch Wärmeeinbringung aus einer externen Wärmequelle (z.B. beheizte Pressvorrichtung) über den jeweiligen Schmelzpunkt erwärmt wird bzw. werden, wodurch Formschicht und Deckschicht aneinander anschmelzen, indem beispielsweise die Formschicht in die Deckschicht eindringt oder diese umschließt. Formschicht und Deckschicht sind somit miteinander verbunden und nach dem Aushärten der angeschmolzenen Formschicht und/oder Deckschicht ist eine feste Verbindung hergestellt.

Unter mechanischer Verbindung werden unter anderem formschlüssige oder kraftschlüssige Verbindungen verstanden, die mit Hilfe von entsprechenden Verbindungsvorrichtungen wie z.B. Nieten oder Stifte hergestellt werden können. Die Verbindungsvorrichtungen können dabei an der Formschicht und/oder der wenigstens einen Deckschicht angeordnet, vorzugsweise einstückig angeformt, sein. Bei den Verbindungsvorrichtungen kann es sich aber auch um gesonderte Verbindungsmittel handeln, mit denen Formschicht und Deckschicht miteinander verbunden werden, z.B. indem sie in einer Pressvorrichtung zusammengepresst werden.

Eine vorgeschlagene Folie umfasst also wenigstens eine Deckschicht und eine formgebende Formschicht, wobei sowohl die wenigstens eine Deckschicht als auch die formgebende Formschicht im Körper bioresorbierbar sind.

Die wenigstens eine Deckschicht, die beispielsweise als Membran ausgebildet sein kann, kann der Überdeckung und Abdichtung einer Knochendefektstelle dienen, um das Eindringen von Weichgewebe in die Knochendefektstelle zu vermeiden. Sie kann darüber hinaus flexibel und vorzugsweise elastisch sein, um eine gute Abdeckung und Abdichtung der Knochendefektstelle zu ermöglichen. Um die Anbringung der Folie und die Abdichtung der Knochendefektstelle weiter zu verbessern, kann die wenigstens eine Deckschicht auch so ausgebildet sein, dass sie mit einem die Knochendefektstelle umgebenden Zahnfleisch verklebt.

Die formgebende Formschicht kann als im Wesentlichen formstabile Schicht ausgebildet sein, die sich sowohl unter thermischem als auch mechanischem und/oder chemischem Einfluss verformen lässt und nach dieser Verformung wiederum eine ausreichende Formstabilität aufweist, um den für das Knochenwachstum zu bildenden Hohlraum für die erforderliche Zeitspanne aufrecht zu erhalten. Die Formschicht kann insbesondere der Anformung der Folie an eine Knochendefektstelle dienen.

Durch die formgebende Formschicht kann ein Hohlraum zwischen Knochendefektstelle und Folie gebildet werden, sodass in diesem Hohlraum Knochenwachstum stattfinden kann. Für eine günstige Knochenregeneration kann der Hohlraum auch Knochenersatzmaterialien und/oder Träger für Medikamente, Wachstumsfaktoren und/oder sonstige die Heilung und Knochenbildung fördernde und schützende Substanzen enthalten. Der Hohlraum kann durch die raumbildende und raumhaltende Formschicht solange aufrecht erhalten werden, bis der Hohlraum durch nachwachsendes Knochenmaterial ausgefüllt ist.

Das vorgeschlagene Verfahren ermöglicht die Herstellung einer vorverbundenen mehrschichtigen Folie umfassend eine formgebende Formschicht und wenigstens eine Deckschicht.

Dadurch, dass sowohl die wenigstens eine Deckschicht als auch die Formschicht bioresorbierbar sind, kann die Folie oder Membran insgesamt vollständig im Körper abgebaut werden (z.B. durch Hydrolyse). Somit entfällt das Durchführen einer weiteren Operation zur Entfernung der Folie. Mit anderen Worten ist hierbei lediglich eine Operation zur Anbringung der Folie erforderlich.

Dadurch, dass die wenigstens eine Deckschicht mit der formgebenden Formschicht als bereits vorverbundene mehrschichtige Folie bereitgestellt wird, ergibt sich eine leicht handhabbare Folie zur Abdeckung einer Knochendefektstelle, welche die raumbildenden Eigenschaften der Formschicht mit den abdichtenden Eigenschaften der Deckschicht verbindet und die darüber hinaus im Körper im Wesentlichen vollständig resorbiert wird.

Die durch das vorgeschlagene Verfahren hergestellte mehrschichtige Folie kann dabei als chirurgische und/oder therapeutische Folie breit eingesetzt werden. So kann eine vorgeschlagene mehrschichtige Folie neben der spezifischen Anwendung im Bereich der Kieferaugmentation auch bei Augenhöhlenbrüchen, Schädelbrüchen sowie allgemein im Bereich der Neurochirurgie und Traumatologie und generell für chirurgische und/oder therapeutische Zwecke eingesetzt werden.

Gemäß einer bevorzugten Ausführungsform der Erfindung kann vorgesehen sein, dass zur thermischen Verbindung der wenigstens einen Deckschicht mit der Formschicht zumindest die Formschicht erwärmt wird. Vorzugsweise kann dabei die Formschicht auf eine Temperatur im Bereich von etwa 50°C bis etwa 70°C, vorzugsweise auf eine Temperatur von etwa 60°C, erwärmt werden. Dadurch, dass die Formschicht thermisch verformbar ist, kann diese durch eine Wärmeeinwirkung von außen thermisch mit der wenigstens einen Deckschicht verbunden werden, indem die Formschicht beispielsweise an die Deckschicht angeschmolzen wird.

Es ist entweder vorgesehen, dass an der Formschicht dornenartige Vorsprünge angeordnet sind, wobei durch das Anlegen der wenigstens einen Deckschicht an die Formschicht die Vorsprünge in die wenigstens eine Deckschicht gedrückt werden und/oder durch die wenigstens eine Deckschicht hindurchgesteckt werden.

Alternativ oder zusätzlich ist vorgesehen, dass vor dem thermischen und/oder mechanischen Verbinden im Wesentlichen vollständig bioresorbierbare Verbindungsvorrichtungen, vorzugsweise Nieten oder Stifte, durch die Formschicht und die wenigstens eine Deckschicht hindurchgesteckt werden. Vorzugsweise sind die Verbindungsvorrichtungen ebenfalls thermisch verformbar. Auch hier können beim Verpressen der Formschicht mit der wenigstens einen Deckschicht - vorzugsweise unter Wärmeeinwirkung von außen - die Enden der Verbindungsvorrichtungen nietenförmig verformt werden und damit eine verbesserte Verbindung der Schichten bewirken, beispielsweis in Form einer Nietverbindung.

Vorzugsweise kann vorgesehen sein, dass die Vorsprünge einstückig an die Formschicht angeformt sind. Durch das Vorsehen von Vorsprüngen an der Formschicht kann eine verbesserte mechanische Verbindung der Formschicht mit der wenigstens einen Deckschicht erzielt werden. Wenn die Formschicht und auch deren Vorsprünge thermisch verformbar sind, können die Vorsprünge durch eine Wärmeeinwirkung von außen thermisch mit der wenigstens einen Deckschicht verbunden werden, indem die Vorsprünge beispielsweise in der Deckschicht eingeschmolzen werden. Wenn die Vorsprünge durch die wenigstens eine Deckschicht hindurchragen, können sich die Enden der Vorsprünge bei der mechanischen und/oder thermischen Verbindung der Formschicht mit der wenigstens einen Deckschicht beispielsweise pilzkopfartig verformen und so eine verbesserte Verbindung der Schichten bewirken.

In einer bevorzugten Ausführungsform kann vorgesehen sein, dass die Enden der dornenartigen Vorsprünge in Form von Widerhaken oder Pilzköpfen ausgebildet sind. Dies verstärkt die Verbindung der Formschicht mit der wenigstens einen Deckschicht, da sich die Widerhaken oder Pilzköpfe der dornenartigen Vorsprünge in der wenigstens einen Deckschicht in der Art eines Klettverschlusses verhaken können.

In einer weiteren Variante kann vorgesehen sein, dass eine erste Deckschicht, eine zweite Deckschicht und eine Formschicht sandwichartig angeordnet werden, wobei die Formschicht zwischen der ersten Deckschicht und der zweiten Deckschicht angeordnet wird. Dabei kann vorgesehen sein, dass die Formschicht eine geringere Flächenausdehnung aufweist als die erste Deckschicht und die zweite Deckschicht. Vorzugsweise kann vorgesehen sein, dass die erste Deckschicht direkt mit der zweiten Deckschicht verbunden, vorzugsweise verwebt, wird. So können erste Deckschicht und zweite Deckschicht an deren äußeren Rändern verwebt werden, wobei die Formschicht innerhalb der äußeren Rändern der ersten Deckschicht und zweiten Deckschicht zwischen erster Deckschicht und zweiter Deckschicht angeordnet ist.

Vorzugsweise kann vorgesehen sein, dass die wenigstens eine Deckschicht mit der Formschicht in einer Pressvorrichtung verpresst wird. Dabei kann vorgesehen sein, dass während des Verpressens wenigstens eine der Folie zugewandte Fläche der Pressvorrichtung erwärmt wird, vorzugsweise auf eine Temperatur im Bereich von etwa 50°C bis etwa 70°C, besonders bevorzugt auf eine Temperatur von etwa 60°C.

Schutz wird auch begehrt für eine mehrschichtige Folie mit den Merkmalen des Anspruchs 5. Vorteilhafte Ausgestaltungen der mehrschichtigen Folie sind in den davon abhängigen Ansprüchen angegeben.

Es ist vorgesehen, dass die wenigstens eine Deckschicht mit einer thermisch verformbaren und im Wesentlichen vollständig bioresorbierbaren Formschicht thermisch und/oder mechanisch verbunden ist.

Es ist entweder vorgesehen, dass an der Formschicht dornenartige Vorsprünge angeordnet sind, wobei vorzugsweise die Vorsprünge einstückig an die Formschicht angeformt sind.

Alternativ oder zusätzlich ist vorgesehen, dass im Wesentlichen vollständig bioresorbierbare Verbindungsvorrichtungen, vorzugsweise Nieten oder Stifte, durch die Formschicht und die wenigstens eine Deckschicht hindurchgesteckt sind.

Gemäß einer bevorzugten Ausführungsform kann vorgesehen sein, dass die Formschicht und die wenigstens eine Deckschicht in unterschiedlichen Zeiträumen im Wesentlichen vollständig bioresorbierbar sind. So kann beispielsweise durch Auslegung der Formschicht und der wenigstens einen Deckschicht erreicht werden, dass die Formschicht schneller resorbiert als die wenigstens eine Deckschicht. Generell ergeben sich durch unterschiedlich starke Bioresorbierbarkeiten der Formschicht und der wenigstens eine Deckschicht große Freiheiten in der Auslegung der Folie in Bezug auf ihre Bioresorbierbarkeit.

Es kann vorgesehen sein, dass die Folie insgesamt in einem Zeitraum von etwa 3 bis 12 Monaten, vorzugsweise etwa 4 bis 6 Monaten, im Wesentlichen vollständig bioresorbierbar ist. Dies ist der Zeitraum innerhalb dessen im Normalfall der Knochenwiederaufbau erfolgt ist.

Um eine gute Anformung an die Knochendefektstelle und eine stabile Hohlraumbildung zwischen Folie und Knochendefektstelle zu ermöglichen, kann vorgesehen sein, dass die Formschicht steifer als die wenigstens eine Deckschicht ausgebildet ist. Die höhere Steifigkeit der Formschicht dient dabei dazu, einen Hohlraum für den Knochenaufbau zu bilden und diesen Hohlraum auch für den für die Knochenregeneration benötigten Zeitraum aufrecht zu erhalten. Durch eine im Vergleich zur Formschicht geringeren Steifigkeit der wenigstens einen Deckschicht kann wiederum eine gute Überdeckung und Abdichtung der Knochendefektstelle erzielt werden.

Vorzugsweise kann vorgesehen sein, dass die Formschicht, gegebenenfalls zusammen mit der wenigstens einen Deckschicht, sowohl thermisch als auch mechanisch und/oder chemisch verformbar ausgebildet ist. So kann insbesondere die Formschicht als im Wesentlichen formstabile Schicht ausgebildet sein, die sich sowohl unter thermischem als auch unter mechanischem oder chemischem Einfluss verformen lässt und nach dieser Verformung wiederum eine ausreichende Formstabilität aufweist, um den für das Knochenwachstum zu bildenden Hohlraum für die erforderliche Zeitspanne aufrecht zu erhalten. Die wenigstens eine Deckschicht kann flexibel und vorzugsweise elastisch sein, um eine gute Abdeckung und Abdichtung der Knochendefektstelle zu ermöglichen.

Eine mechanische Verformung kann dabei beispielsweise durch Verbiegen mit einer Zange erfolgen. Dies ist insbesondere für verhältnismäßig dünne Formschichten (z.B. im Bereich von etwa 0,10 mm bis etwa 0,5 mm) eine geeignete Methode der Anformung. Für dickere Formschichten (z.B. dicker als etwa 0,5 mm) kann eine thermische Verformung einer Formschicht zur Anformung zweckmäßig sein. Eine entsprechende thermische Verformung kann dabei beispielsweise mittels eines Thermostabes mit einer heißen Spitze oder Fläche, über erwärmte vorgefertigte Modelle oder im heißen Wasserbad mit steriler Kochsalzlösung erzielt werden.

Für eine gute Bioresorbierbarkeit der vorgeschlagenen Folie kann vorgesehen sein, dass die wenigstens eine Deckschicht zumindest teilweise, vorzugsweise im Wesentlichen vollständig, aus einem bioresorbierbaren Collagenmaterial besteht. Dabei kann vorgesehen sein, dass das bioresorbierbare Collagenmaterial Typ-I-Collagen und/oder Typ-III-Collagen umfasst. Das Collagenmaterial kann beispielsweise aus bovinen Achillessehnen stammen.

Es kann auch vorgesehen sein, dass die wenigstens eine Deckschicht zumindest teilweise aus poly(lactic-co-glycolic acid)-polyethylene glycol-poly(lactic-co-glycolic acid) besteht. Dieses Material ist im Handel beispielsweise unter der Bezeichnung "vicryl mesh" erhältlich.

Es kann vorgesehen sein, dass die wenigstens eine Deckschicht als eine Membran ausgebildet ist, die zumindest teilweise aus Fibrin besteht.

Des Weiteren kann vorgesehen sein, dass die wenigstens eine Deckschicht als eine lyophilisierte Membran ausgebildet ist, die zumindest teilweise aus Rinder-Pericard oder Rinder-Dura Mater besteht.

Für eine gute Bioresorbierbarkeit der vorgeschlagenen Folie kann darüber hinaus vorgesehen sein, dass die Formschicht zumindest teilweise, vorzugsweise im Wesentlichen vollständig, aus einem bioresorbierbaren Polymermaterial besteht. Bei dem bioresorbierbaren Polymermaterial kann es sich auch um ein Co-Polymermaterial handeln.

Eine besondere Ausführungsvariante sieht vor, dass das bioresorbierbare Polymermaterial Milchsäure, vorzugsweise L-Milchsäure, und/oder deren Derivate umfasst. Vorteilhaft ist es dabei, wenn der Anteil der Milchsäure im bioresorbierbaren Polymermaterial mindestens 70%, vorzugsweise etwa 80% bis 95%, besonders bevorzugt im Wesentlichen etwa 82%, beträgt.

Darüber hinaus kann vorgesehen sein, dass das bioresorbierbare Polymermaterial Glykolsäure umfasst. Vorteilhaft ist es dabei, wenn der Anteil der Glykolsäure im bioresorbierbaren Polymermaterial höchstens 30%, vorzugsweise etwa 15% bis 20%, besonders bevorzugt im Wesentlichen etwa 18%, beträgt. Je nach Zusammensetzung der Formschicht kann erreicht werden, dass die Formschicht im Wesentlichen formstabil und dennoch im Wesentlichen vollständig bioresorbierbar ist.

Es kann vorgesehen sein, dass die Formschicht zumindest teilweise einen Polyester aus der Familie der Poly-α-Hydroxyl-Säuren, wie Polytrimethylencarbonat, Polydioxanon, Polyglycolid, Polylactid, Poly(L-lactid-co-glycolid) nebst anderen Copolymeren, Polyorthoester und/oder Polycaprolacton (Polyhydroxybutyrat und Polyhydroxybutyrat-co-hydroxyvalerat) aufweist.

Es kann auch vorgesehen sein, dass die Formschicht zumindest teilweise aus Poly(D,L-lactid) besteht.

Es kann auch vorgesehen sein, dass die Formschicht zumindest teilweise aus Poly(L-lactid-co-D,L-lactid) besteht.

Bei einer weiteren bevorzugten Ausführungsform kann vorgesehen sein, dass die Formschicht und die wenigstens eine Deckschicht unterschiedliche Flächenausdehnungen aufweisen. Dabei kann vorgesehen sein, dass die Formschicht eine geringere Flächenausdehnung als die wenigstens eine Deckschicht einnimmt. Wenn die wenigstens eine Deckschicht die Formschicht aufgrund ihrer geringeren Flächenausdehnung überdeckt, kann eine besonders gute Abdeckung und somit auch Abdichtung der Knochendefektstelle erreicht werden.

Vorzugsweise kann vorgesehen sein, dass die wenigstens eine Deckschicht und/oder die Formschicht im Wesentlichen durchgehend flächig ausgebildet ist bzw. sind. Eine für die Anformung an die Knochendefektstelle günstige Kontur der Folie kann dabei beispielsweise durch entsprechendes Schneiden der Folie erreicht werden.

Besonders günstig ist es jedoch, wenn die Formschicht zur Anformung an die Knochendefektstelle eine Formstruktur aufweist. Dabei kann vorgesehen sein, dass die Formstruktur wenigstens abschnittsweise einen konvex und/oder konkav gekrümmten Rand und/oder wenigstens abschnittsweise eine konvex und/oder konkav gekrümmte Form aufweist. Mit anderen Worten kann die Formstruktur beispielsweise flächige - konvex und/oder konkav gekrümmte - Vorsprünge aufweisen und somit einen konvex und/oder konkav gekrümmten Rand aufweisen. Alternativ oder zusätzlich kann auch die Formstruktur als ganzes eine entsprechend konvex und/oder konkav gekrümmte Form aufweisen.

Besonders vorteilhaft ist es, wenn die Formstruktur wenigstens ein strebenförmiges Anformelement aufweist. Die strebenförmigen oder lappenförmigen Anformelemente können dabei bügelartig über die Knochendefektstelle geformt werden und eine beliebige Hohlraumform ermöglichen.

Besonders vorteilhaft ist jene Ausführungsform der Erfindung, bei der die Formstruktur im Wesentlichen gitterförmig ausgebildet ist. Die gitterförmige Formstruktur bildet in diesem Fall ein Verstärkungsgitter, das die Bildung einer Vielzahl von beliebigen Hohlraumformen ermöglicht.

Es kann auch vorgesehen sein, dass die Formstruktur durch wenigstens eine Verstärkung der Formschicht ausgebildet ist. Insbesondere, wenn die Formschicht in Form einer aushärtenden Flüssigkeit oder eines aushärtenden Gels auf die wenigstens eine Deckschicht aufgebracht wird, ist es günstig, wenn die Formstruktur lediglich durch das Aufbringen von mehr Flüssigkeit oder Gel im Bereich der Formstruktur erzielt werden kann. In diesem Fall kann beispielsweise die Formschicht unterschiedliche Dicken aufweisen.

Eine besondere Ausführungsvariante sieht vor, dass die Folie eine Trägerschicht für wenigstens eine darauf anzuordnende oder angeordnete Substanz aufweist. Bei den auf der Trägerschicht anzuordnenden oder angeordneten Substanzen kann es sich dabei um Medikamente, Wachstumsfaktoren und/oder sonstige die Heilung und Knochenbildung fördernde und schützende Substanzen handeln. Die Trägerschicht kann vorzugsweise an einer der Knochendefektstelle zuzuwendenden Seite der Folie angeordnet sein und zumindest teilweise, vorzugsweise im Wesentlichen vollständig, aus einem bioresorbierbaren Collagenmaterial bestehen.

Es kann auch vorgesehen sein, dass entsprechende Substanzen direkt auf der Formschicht und/oder der wenigstens einen Deckschicht angebracht sind. Es kann auch vorgesehen sein, dass die einer Knochendefektstelle zuzuwendende Seite bzw. Oberfläche der Folie selbst als Träger für die oben beschriebenen Substanzen dient, indem beispielsweise diese Seite bzw. Oberfläche der Folie eine entsprechende Rauigkeit aufweist.

Die vorgeschlagene Folie oder Membran kann je nach Anwendungsfall auch vorgeschnitten und/oder vorgeformt bereitgestellt werden. Dabei kann beispielsweise ein gewünschter Zuschnitt und/oder eine gewünschte 3D-Verformung der Folie nach einer datenverarbeitungsgestützten Planung erfolgen.

Weitere Einzelheiten und Vorteile der vorliegenden Erfindung werden anhand der nachfolgenden Figurenbeschreibung erläutert. Dabei zeigen:
- Fig. 1: eine schematische Pressvorrichtung zur Herstellung einer vorgeschlagenen mehrschichtigen Folie,
- Fig. 2: eine gemäß dem vorgeschlagenen Verfahren hergestellte mehrschichtige Folie in einer schematischen Seitenansicht,
- Fig. 3a - 3c: ein Ausführungsbeispiel der Herstellung einer vorgeschlagenen mehrschichtigen Folie mit einer Formschicht mit angeformten, dornenartigen Vorsprüngen,
- Fig. 4a - 4c: ein weiteres Ausführungsbeispiel der Herstellung einer vorgeschlagenen mehrschichtigen Folie mit einer Formschicht mit angeformten, dornenartigen Vorsprüngen,
- Fig. 5a - 5d: ein Ausführungsbeispiel der Herstellung einer vorgeschlagenen mehrschichtigen Folie unter Einsatz von nietartigen Verbindungsvorrichtungen,
- Fig. 6: ein Ausführungsbeispiel der vorgeschlagenen mehrschichtigen Folie in einer perspektivischen Explosionsdarstellung,
- Fig. 7: eine Seitenansicht der vorgeschlagenen mehrschichtigen Folie gemäß Figur 6,
- Fig. 8: eine Draufsicht auf ein weiteres Ausführungsbeispiel der vorgeschlagenen mehrschichtigen Folie,
- Fig. 9: eine Formschicht mit angeformten, dornenartigen Vorsprüngen in perspektivischer Ansicht,
- Fig. 10: eine Formschicht mit daran angeordneten stiftförmigen Vorsprüngen in perspektivischer Ansicht,
- Fig. 11: ein Ausführungsbeispiel der vorgeschlagenen mehrschichtigen Folie in einer perspektivischen Explosionsdarstellung,
- Fig. 12: die Folie gemäß Fig. 11 nach dem Anlegen der Deckschichten an die Formschicht,
- Fig. 13: die Folie gemäß Fig. 12 nach einem Verpressen der Deckschichten mit der Formschicht,
- Fig. 14: die fertig hergestellte Folie gemäß Fig. 13 in einer Seitenansicht,
- Fig. 15: eine Deckschicht mit Ausnehmungen für Vorsprünge der Formschicht,
- Fig. 16: eine weitere Deckschicht mit Ausnehmungen für Vorsprünge der Formschicht,
- Fig. 17: eine Draufsicht auf ein weiteres Ausführungsbeispiel der vorgeschlagenen mehrschichtigen Folie
- Fig. 18 - 21: Draufsichten auf verschiedene weitere Ausführungsformen der vorgeschlagenen mehrschichtigen Folie,
- Fig. 22 - 29: mehrere Ausführungsbeispiele der vorgeschlagenen mehrschichtigen Folie in perspektivischen Explosionsdarstellungen,
- Fig. 30: eine vorgeschlagene mehrschichtige Folie angeordnet an einer Knochendefektstelle eines Kieferknochens,
- Fig. 31: eine vorgeschlagene mehrschichtige Folie angeordnet an einer Knochendefektstelle eines Kieferknochens mit Implantat,
- Fig. 32: eine vorgeschlagene mehrschichtige Folie angeordnet an einer Knochendefektstelle eines Kieferknochens mit Implantat, Beilagscheibe und Pfosten,
- Fig. 33: eine vorgeschlagene mehrschichtige Folie eingeklemmt in einer Nut einer Beilagscheibe,
- Fig. 34: eine vorgeschlagene mehrschichtige Folie eingeklemmt in einer Nut einer Beilagscheibe und angeordnet an einer Knochendefektstelle eines Kieferknochens mit Implantat und Pfosten,
- Fig. 35: eine vorgeschlagene mehrschichtige Folie angeordnet an einer Knochendefektstelle um einen natürlichen Zahn und
- Fig. 36: eine vorgeschlagene mehrschichtige Folie angeordnet an einer Knochendefektstelle um einen natürlichen Zahn in einer Schnittdarstellung.

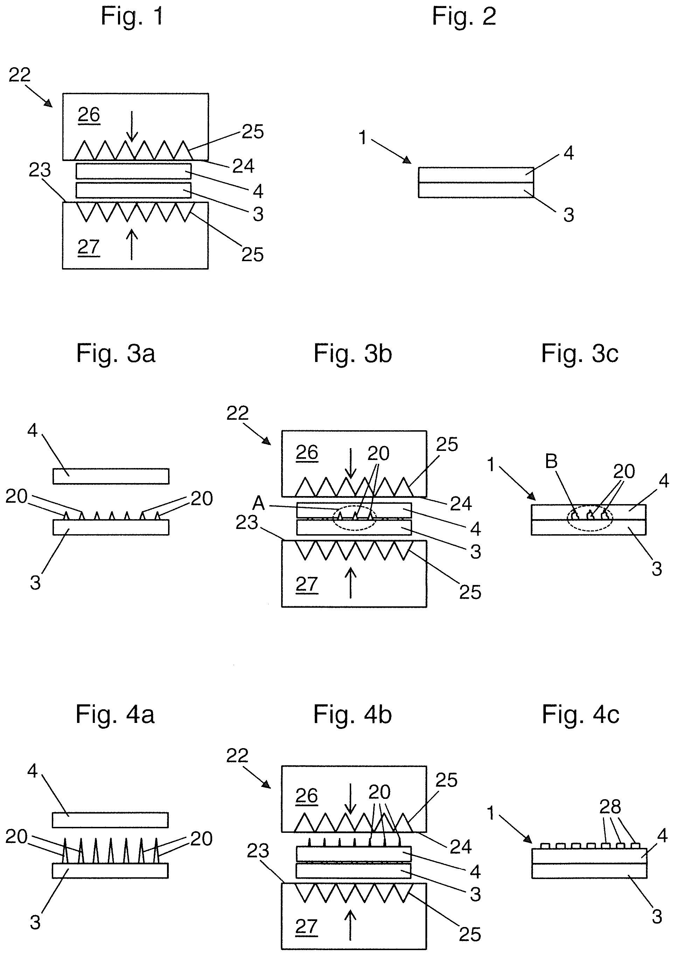

Fig. 1 zeigt schematisch eine Pressvorrichtung 22 zur Herstellung einer vorgeschlagenen mehrschichtigen Folie 1 in einer Seitenansicht. Die Pressvorrichtung 22 weist zwei Pressbacken 26, 27 mit Pressflächen 23, 24 auf, zwischen denen eine Deckschicht 4 und eine Formschicht 3 angeordnet sind. Sowohl Deckschicht 4 als auch Formschicht 3 sind bioresorbierbar. Die Formschicht 3 ist darüber hinaus thermisch verformbar, das heißt, dass sich die Formschicht 3 unter Wärmeeinwirkung von außen verformen lässt.

Die Pressvorrichtung 22 ist mit wenigstens einer Heizvorrichtung 25 ausgestattet, die es ermöglicht, dass die der Folie 1 zugewandten Flächen 23 und 24 der Pressvorrichtung 22 erwärmt werden können, wodurch sich in weiterer Folge die Folie 1 oder zumindest die thermisch verformbare Formschicht 3 der Folie 1 erwärmen lässt. Durch Bewegen von Pressbacken 26, 27 der Pressvorrichtung 22 in Pfeilrichtung wird die an die Formschicht 3 angelegte Deckschicht 4 mit der Formschicht 3 thermisch und/oder mechanisch verbunden, vorzugsweise verpresst. Durch die wenigstens eine Heizvorrichtung 25 können die Flächen 23, 24 der Pressbacken 26, 27 der Pressvorrichtung 22 auf dieselbe Temperatur oder auch auf unterschiedliche Temperaturen erwärmt werden.

Fig. 2 zeigt die Folie 1 gemäß Fig. 1 nach dem Verbinden durch die Pressvorrichtung 22. Die Deckschicht 4 und die Formschicht 3 sind fest miteinander verbunden und bilden eine verbundene mehrschichtige Folie 1.

Die Fig. 3a bis 3c zeigen die Herstellung einer weiteren vorgeschlagenen, mehrschichtigen Folie 1. Fig. 3a zeigt zwei Schichten der herzustellenden Folie 1, nämlich eine Deckschicht 4 und eine Formschicht 3. Die Formschicht 3 weist dornenartige Vorsprünge 20 auf, die in diesem Beispiel einstückig an die Formschicht 3 angeformt sind. Beim oder durch das Anlegen der Deckschicht 4 an die Formschicht 3 bohren sich die Vorsprünge 20 in die Deckschicht 4, ragen in diesem Beispiel jedoch nicht durch die Deckschicht 4 hindurch. Fig. 3b zeigt die Anordnung von Deckschicht 4 und Formschicht 3 nach dem Anlegen der Deckschicht 4 an die Formschicht 3. In einer Pressvorrichtung 22 mit Heizvorrichtung 25 erfolgt nun eine mechanische und thermische Verbindung der Deckschicht 4 mit der Formschicht 3, indem die Pressbacken 26, 27 der Pressvorrichtung 22 in Pfeilrichtung aufeinander zubewegt werden und dabei die Deckschicht 4 mit der Formschicht 3 verpressen. Durch Erwärmen einer Fläche 23 der Pressbacke 27 und/oder einer Fläche 24 der Pressbacke 26 mittels Heizvorrichtung 25 kann während dieses Verpressens die Formschicht 3 erwärmt werden, beispielsweise auf eine Temperatur im Bereich von etwa 50 °C bis etwa 70 °C. Durch diese Wärmeeinwirkung von außen kann es zu einem Schmelzen der Vorsprünge 20 kommen, wodurch die Vorsprünge 20 der Formschicht 3 mit der Deckschicht 4 verschmelzen können, wie es in Fig. 3c dargestellt ist. Die in Fig. 3b und 3c markierten Bereiche A, B zeigen jeweils einen aufgebrochenen Bereich der Folie 1, um darzustellen, wie die Vorsprünge 20 innerhalb der Folie 1 in die Deckschicht 4 eindringen und mit der Deckschicht 4 verschmelzen. Neben einem ebenfalls möglichen Anschmelzen der Formschicht 3 an die Deckschicht 4 ermöglichen die in die Deckschicht 4 eingeschmolzenen Vorsprünge 20 eine feste, dübelartige Verbindung.

Fig. 4a bis 4c zeigt ein weiteres Beispiel eines Herstellungsverfahrens einer vorgeschlagenen Folie 1. Ähnlich wie in Fig. 3a weist auch hier die Formschicht 3 Vorsprünge 20 auf, die in diesem Beispiel jedoch so lang sind, dass sie beim Anlegen der Deckschicht 4 an die Formschicht 3 durch die Deckschicht 4 hindurchragen, wie es in Fig. 4b dargestellt ist. Durch Verpressen der Deckschicht 4 mit der Formschicht 3 mittels Pressvorrichtung 22 verformen sich die durch die Deckschicht 4 hindurchragenden Spitzen der Vorsprünge 20 pilzkopfartig, sodass eine fest verbundene mehrschichtige Folie 1 hergestellt wird, wie in Fig. 4c dargestellt. Zusätzlich zu einer Verbindung der aneinander anliegenden Oberflächen von Deckschicht 4 und Formschicht 3 sorgen die durch das thermische Verpressen geformten Köpfe 28 der Vorsprünge 20 für eine formschlüssige Verbindung von Deckschicht 4 mit Formschicht 3.

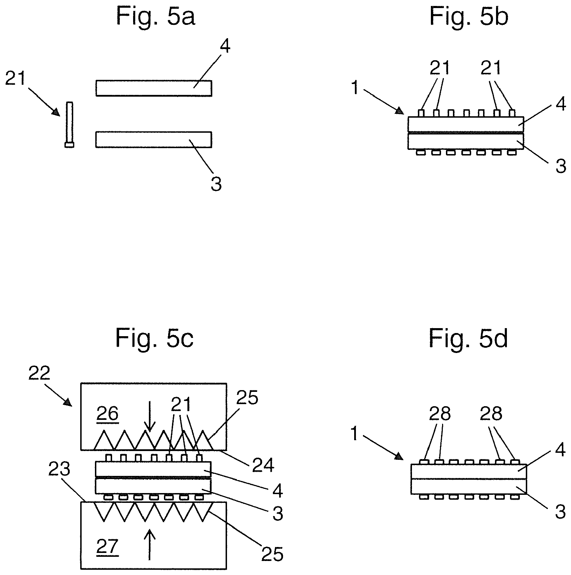

Fig. 5a bis 5d zeigt ein weiteres Beispiel der Herstellung einer vorgeschlagenen mehrschichtigen Folie 1. Hierbei kommen gemäß Fig. 5a neben einer Deckschicht 4 und einer Formschicht 3 mehrere Verbindungsvorrichtungen 21 in Form von Nieten oder Stiften zum Einsatz. Fig. 5b zeigt die an die Formschicht 3 angelegte Deckschicht 4 sowie eine Mehrzahl der Verbindungsvorrichtungen 21, die durch Formschicht 3 und Deckschicht 4 hindurchgesteckt wurden. Die Verbindungsvorrichtungen 21 sind in diesem Beispiel an einem Ende mit einem Kopf ausgestattet, wobei die Köpfe an der Formschicht 3 anliegen und die freien Enden der Verbindungsvorrichtungen 21 durch Formschicht 3 und Deckschicht 4 hindurchragen und die Deckschicht 4 überragen. Gemäß Fig. 5c wird die Anordnung der Fig. 5b in eine Pressvorrichtung 22 eingeführt und mittels Pressbacken 26, 27 der Pressvorrichtung 22 thermisch verpresst. Fig. 5d zeigt die fertig hergestellte mehrschichtige Folie 1. Hierbei ist zu erkennen, dass durch das Verpressen die freien Enden der Verbindungsvorrichtungen 21 derart verformt wurden, dass sich Köpfe 28 bildeten, die an der Deckschicht 4 anliegen. Dadurch ergibt sich eine feste formschlüssige Verbindung in der Art einer Nietverbindung.

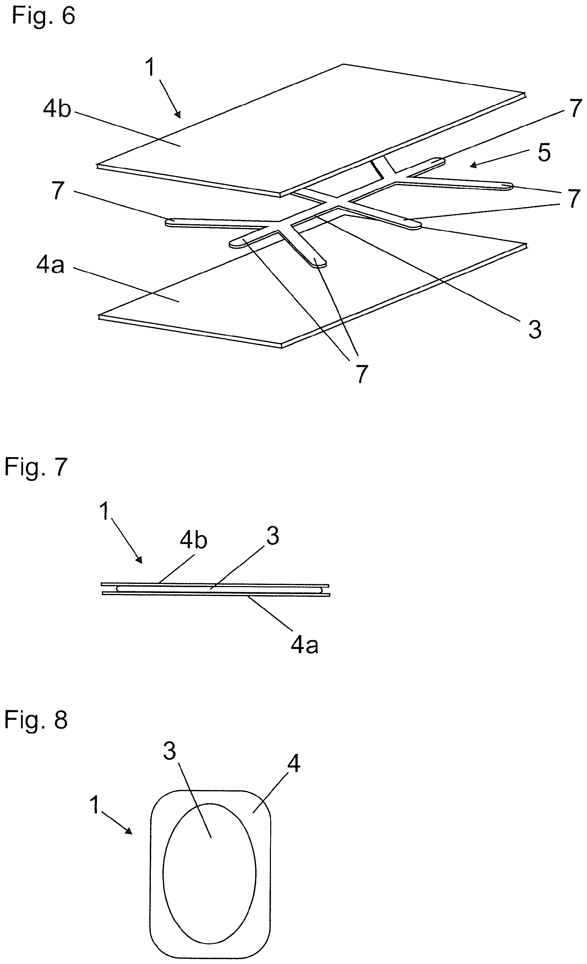

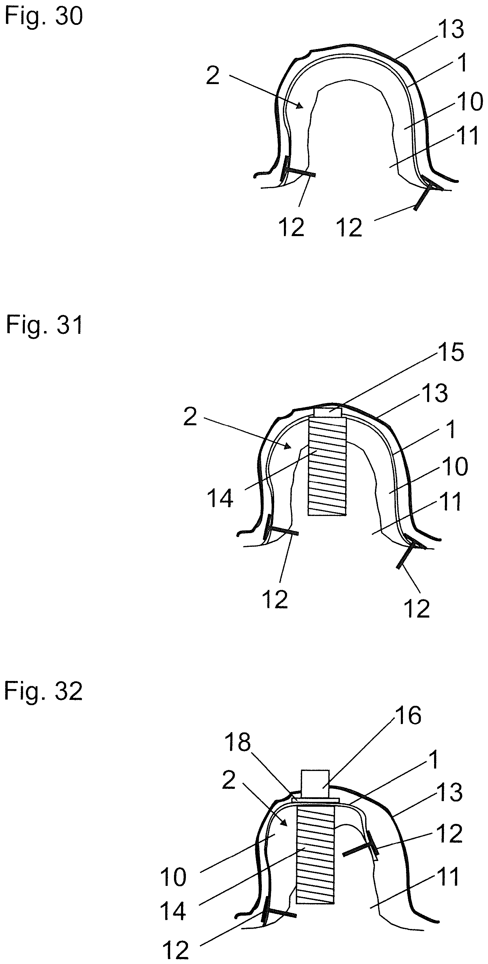

Figur 6 zeigt eine perspektivische Explosionsdarstellung einer vorgeschlagenen vorverbundenen mehrschichtigen Folie 1. Die Folie 1 umfasst eine Formschicht 3 sowie zwei Deckschichten 4a und 4b. Die Formschicht 3 ist steifer als die Deckschichten 4a und 4b ausgebildet und weist eine Formstruktur 5 auf. Die Formstruktur 5 umfasst mehrere strebenförmige Anformelemente 7, die dazu dienen, die Folie 1 über eine Knochendefektstelle 2 (hier nicht gezeigt) zu formen, wobei sich die Folie 1 durch die Anformelemente 7 gut an einen noch vorhandenen Knochen 11 der Knochendefektstelle 2 (siehe z.B. Figur 30) anformen lässt. Die Formstruktur 5 ist insgesamt im Wesentlichen gitterförmig ausgebildet und ermöglicht somit die Ausbildung beliebiger Oberflächenformen der Folie 1, sodass sich in Verbindung mit einer Knochendefektstelle 2 beliebige Hohlraumformen zwischen Folie 1 und Knochendefektstelle 2 bilden lassen. Im gezeigten Beispiel weist die Formstruktur 5 ein längserstrecktes Anformelement 7 auf, von dem weitere Anformelemente 7 astförmig abstehen. Durch die astförmig abstehenden Anformelement 7 lässt sich die Formstruktur 5 sehr gut an einen noch vorhandenen Knochen 11 der Knochendefektstelle 2 anformen.

Die Formschicht 3 sowie die Deckschichten 4a und 4b bestehen jeweils aus einem bioresorbierbaren Material, sodass die Folie 1 insgesamt im Körper im Wesentlichen vollständig bioresorbierbar ist. Durch das Vorsehen von zwei Deckschichten 4a und 4b, zwischen denen die Formschicht 3 eingebettet ist, lässt sich insbesondere die Resorptionsgeschwindigkeit und mechanische Festigkeit der Formschicht 3 steuern.

Bei den Deckschichten 4a und 4b kann es sich beispielsweise um bioresorbierbare Collagen-Membranen handeln, die einerseits durch ihre Weichheit eine Knochendefektstelle 2 gut abdecken können und andererseits mit einem die Knochendefektstelle 2 umgebenden Zahnfleisch 13 gut verkleben können, sodass sich eine gute Abdichtung der Knochendefektstelle 2 ergibt.

Die Formschicht 3 kann beispielsweise aus einem bioresorbierbaren Polymermaterial oder Co-Polymermaterial bestehen. Insbesondere kann die Formschicht 3 beispielsweise etwa 82% L-Milchsäure und etwa 18% Glycolsäure umfassen. Eine solche Materialwahl ergibt eine im Wesentlichen formstabile Formschicht 3, die zur Anformung an eine Knochendefektstelle 2 sowohl thermisch als auch mechanisch und/oder chemisch verformbar ausgebildet sein kann, wobei die Formschicht 3 nach einer solchen Verformung im Wesentlichen wiederum formstabil ist. Aufgrund der Steifigkeit und Formstabilität der Formschicht 3 kann somit zwischen der Folie 1 und einer Knochendefektstelle 2 ein Hohlraum für die Knochenregeneration geschaffen und für den Zeitraum der Knochenregeneration auch gehalten werden.

Figur 7 zeigt eine Seitenansicht der verbundenen mehrschichtigen Folie 1 gemäß Figur 6.

Figur 8 zeigt eine Draufsicht auf eine weitere Variante der vorgeschlagenen Folie 1, die in diesem Beispiel zweischichtig ausgeführt ist und eine Formschicht 3 und eine Deckschicht 4 umfasst. Sowohl die Formschicht 3 als auch die Deckschicht 4 sind im Wesentlichen flächig ausgebildet. Die Folie 1 kann beliebig zugeschnitten werden, um je nach Anwendungsfall eine gute Anformung an eine Knochendefektstelle 2 zu ermöglichen.

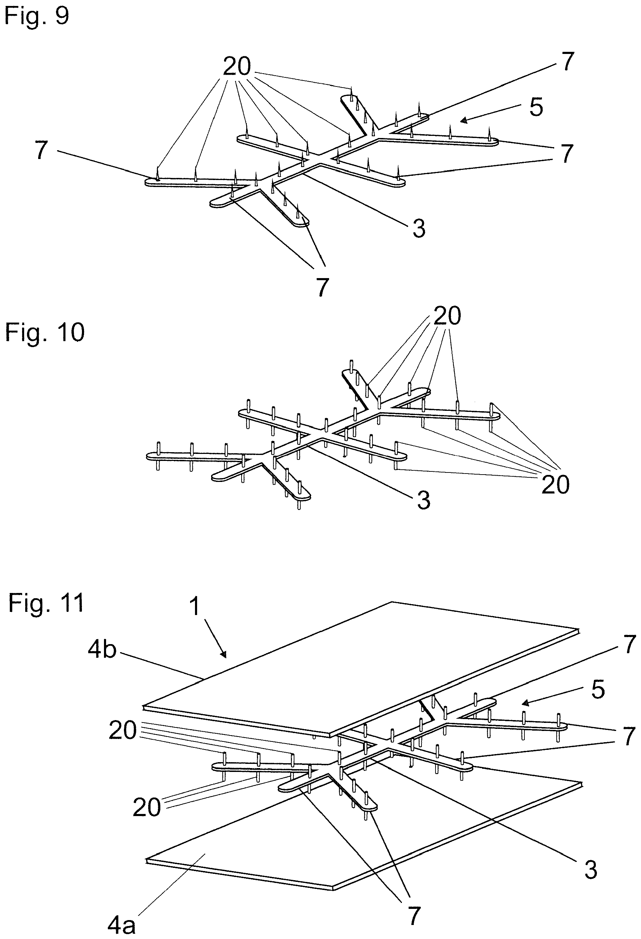

Fig. 9 zeigt ein Beispiel einer Formschicht 3 mit daran angeformten Vorsprüngen 20 und Fig. 10 zeigt ein weiteres Beispiel einer Formschicht 3, wobei in diesem Beispiel sowohl an der Ober- als auch an der Unterseite der Formschicht 3 stiftförmige Vorsprünge 20 angeordnet sind.

Fig. 11 zeigt eine perspektivische Explosionsdarstellung einer vorgeschlagenen mehrschichtigen Folie 1, die eine Formschicht 3 gemäß Fig. 10 sowie zwei Deckschichten 4a und 4b umfasst.

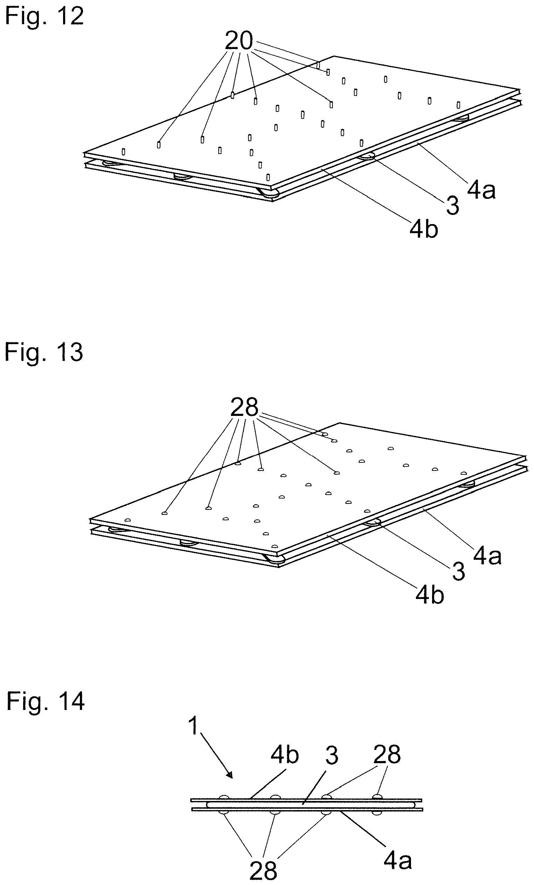

Fig. 12 zeigt die Folie 1 der Fig. 11 nach dem Anlegen der Deckschichten 4a und 4b an die Formschicht 3. Es ist zu erkennen, dass die Vorsprünge 20 der Formschicht 3 durch die Deckschichten 4a und 4b hindurchragen.

Fig. 13 zeigt die Folie 1 der Fig. 12 nach dem Verpressen der Deckschichten 4a und 4b mit der Formschicht 3. Durch das Verpressen, welches mechanisch und/oder thermisch erfolgen kann, wurde eine festverbundene mehrschichtige Folie 1 hergestellt. Die über die Deckschichten 4a, 4b hinausragenden Vorsprünge 20 der Formschicht 3 wurden durch das Verpressen verformt, sodass sich nietförmige Köpfe 28 bildeten, die eine formschlüssige Verbindung von Formschicht 3 mit den Deckschichten 4a, 4b ermöglichen.

Fig. 14 zeigt eine Seitenansicht der hergestellten Folie 1 gemäß Fig. 13.

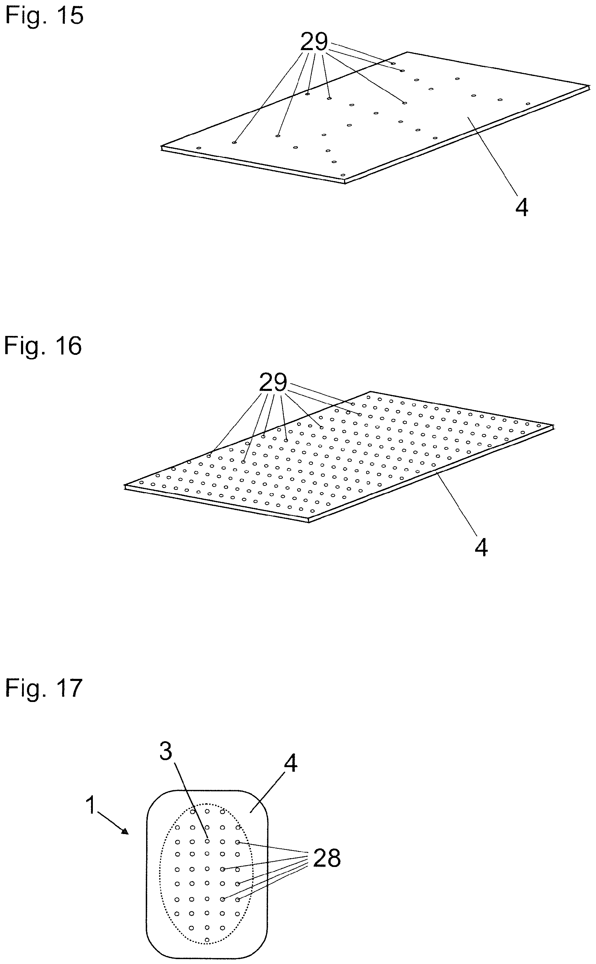

Fig. 15 zeigt eine Deckschicht 4, die insbesondere zur Verbindung mit einer Formschicht 3 gemäß Fig. 9 geeignet ist. Diese Deckschicht 4 weist Ausnehmungen 29 in Form von Löchern auf, die mit den Vorsprüngen 20 der Formschicht 3 korrespondieren, sodass ein passgenaues Anlegen der Deckschicht 4 an die Formschicht 3 ermöglicht wird.

Fig. 16 zeigt ein weiters Beispiel einer Deckschicht 4, die in diesem Fall vollflächig mit entsprechenden Ausnehmungen 29 versehen ist, sodass sich diese Deckschicht 4 beliebig auf Vorsprünge 20 der Formschicht 3 aufsetzen lässt.

Fig. 17 zeigt ein weiteres Beispiel einer vorgeschlagenen Folie 1 in einer Draufsicht. Die Deckschicht 4 ist in diesem Beispiel mit einem Muster von Ausnehmungen 29 versehen, durch die Vorsprünge 20 einer - in diesem Beispiel von der Deckschicht 4 verdeckten - Formschicht 3 hindurchragten, wobei sich die Enden der Vorsprünge 20 beim thermischen und/oder mechanischen Verbinden der Deckschicht 4 mit der Formschicht 3 zu Köpfen 28 verformten.

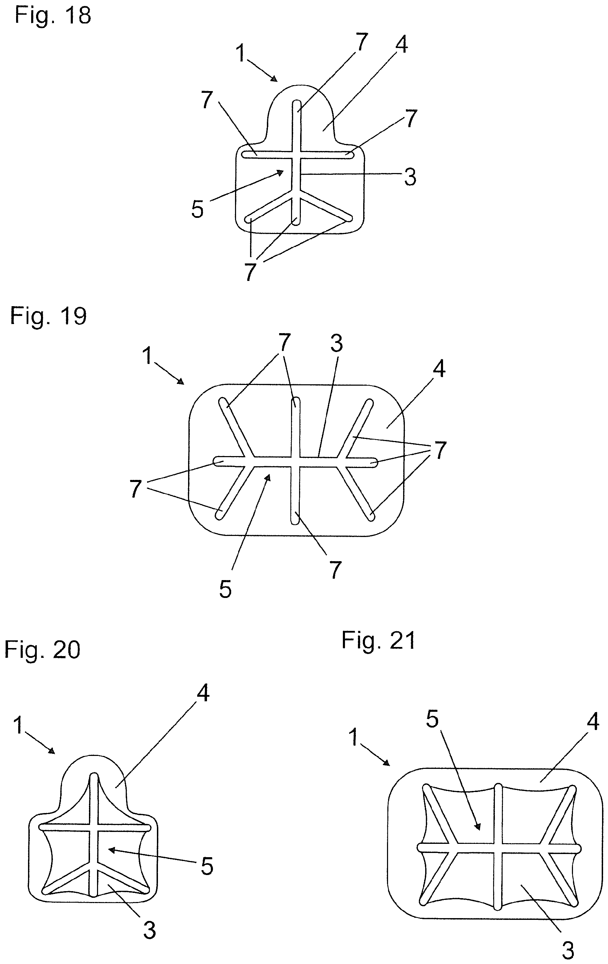

Figur 18 und Figur 19 zeigen zwei weitere Ausführungsformen einer vorgeschlagenen zweischichtigen Folie 1 mit unterschiedlichen äußeren Konturen der Deckschicht 4 und unterschiedlich ausgebildeten Formstrukturen 5 der Formschicht 3.

Figur 20 und Figur 21 zeigen weitere Beispiele von vorgeschlagenen Folien 1, wobei in den hier gezeigten Beispielen die Formschicht 3 jeweils als Gel auf die Deckschicht 4 aufgebracht wurde und in weiterer Folge aushärtete. Die hier gezeigten Formschichten 3 umfassen jeweils eine Formstruktur 5, die beispielsweise dadurch erzielt wurde, dass in den Bereichen der Formstruktur 5 mehr Gel aufgebracht wurde, sodass die Formschichten 3 unterschiedliche Schichtdicken aufweisen. Im Bereich einer Formstruktur 5 weist eine Formschicht 3 eine jeweils stärkere Schichtdicke auf als in den übrigen Bereichen der Formschicht 3.

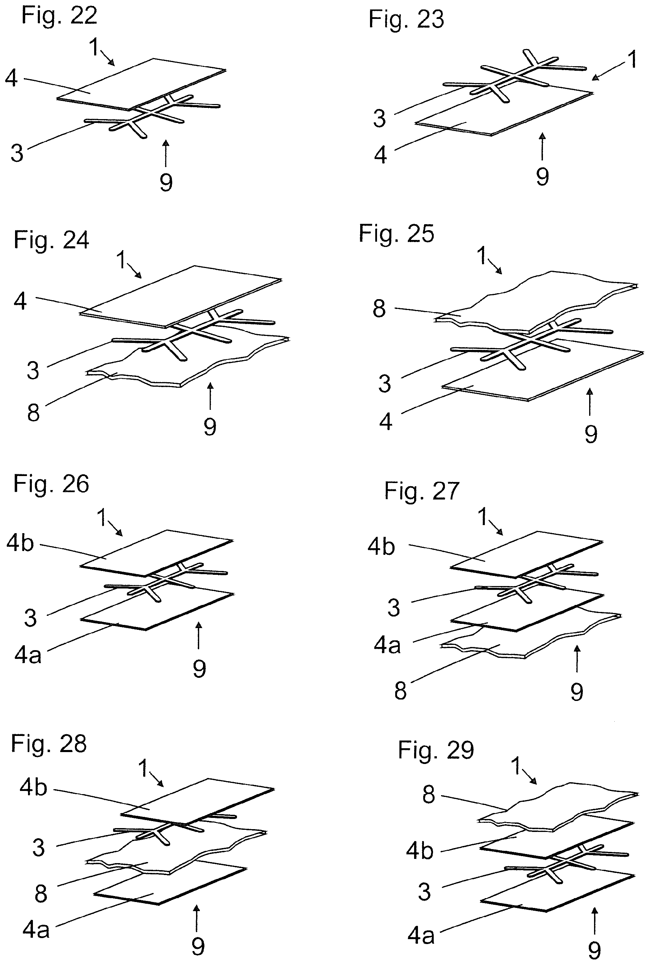

Figur 22 bis Figur 29 zeigen weitere Ausführungsbeispiele einer vorgeschlagenen Folie 1 in jeweils perspektivischer Explosionsdarstellung. Die in den Figuren jeweils nach unten weisende Seite 9 einer Folie 1 ist dabei die einer Knochendefektstelle 2 zuzuwendende Seite 9 der Folie 1.

Die Beispiele der Figur 22 und Figur 23 sind zweischichtig aufgebaut und umfassen jeweils eine Formschicht 3 und eine Deckschicht 4, wobei die Formschicht 3 eine geringere Flächenausdehnung als die Deckschicht 4 einnimmt. Die Beispiele der Figur 24 und Figur 25 sind dreischichtig aufgebaut und umfassen jeweils neben einer Formschicht 3 und einer Deckschicht 4 eine Trägerschicht 8, auf der Substanzen wie beispielsweise Medikamente, Wachstumsfaktoren und/oder sonstige die Heilung und Knochenbildung fördernde und schützende Substanzen aufgebracht sein können.

Die Beispiele der Figuren 26 bis Figur 29 weisen jeweils eine Formschicht 3 und jeweils zwei Deckschichten 4a und 4b auf, wobei die Formschicht 3 eine geringere Flächenausdehnung als die Deckschichten 4a und 4b einnimmt. Die Beispiele der Figur 27 bis Figur 29 weisen jeweils zusätzlich eine Trägerschicht 8 auf, die mit entsprechenden Substanzen (wie oben zu Figur 24 und Figur 25 beschrieben) bestückt sein kann.

Figur 30 zeigt eine Schnittdarstellung eines Kieferknochens 11 mit einer Knochendefektstelle 2. Um einen Knochenaufbau an der Knochendefektstelle 2 zu ermöglichen, wird eine vorgeschlagene mehrschichtige Folie 1 über die Knochendefektstelle 2 entsprechend angeformt und mittels entsprechender Befestigungsvorrichtungen 12 am Kieferknochen 11 verankert. Bei den Befestigungsvorrichtungen 12 kann es sich beispielsweise um bioresorbierbare Nägel handeln. Im sich bildenden Hohlraum 10 zwischen Folie 1 und Knochendefektstelle 2 bzw. Kieferknochen 11 können Knochenersatzmaterialien und/oder Träger für Medikamente, Wachstumsfaktoren und/oder sonstige die Heilung und Knochenbildung und Ausbildung eines Zahnhalteapparates um natürliche Zähne fördernde und schützende Substanzen enthalten sein, um die Knochenregeneration zu fördern. Nach Anbringung der Folie 1 wird das zuvor entfernte oder zurückgeklappte Zahnfleisch 13 wieder über die Folie 1 gedeckt und entsprechend vernäht. Aufgrund der Bioresorbierbarkeit der Folie 1 und der Befestigungsvorrichtungen 12 ist keine weitere Operation nötig, um die Folie 1 und/oder die Befestigungsvorrichtungen 12 nach erfolgtem Knochenaufbau wieder zu entfernen.

Figur 31 zeigt eine Knochendefektstelle 2 ähnlich der aus Figur 30, die mit einer vorgeschlagenen Folie 1 abgedeckt ist. In diesem Beispiel wurde in den noch vorhandenen Kieferknochen 11 ein Implantat 14 eingesetzt, dessen freies Ende mit einer Schraube 15 ausgestattet ist. Für eine erleichterte Zugänglichkeit zum Implantat 14 bzw. dessen Schraube 15 kann in diesem Fall vorgesehen sein, dass die Folie 1 bereits mit einem vorab ausgestanzten Loch versehen ist, durch das die Schraube 15 hindurchragen kann.

Figur 32 zeigt ein weiteres Beispiel einer Knochendefektstelle 2, die mit einer vorgeschlagenen Folie 1 abgedeckt ist. Im Kieferknochen 11 ist bereits ein Implantat 14 eingesetzt, an dessen freiem Ende ein Pfosten 16 angeordnet ist. Der Pfosten 16 ragt sowohl durch die Folie 1, als auch durch das Zahnfleisch 13 hindurch, um einen weiteren Zahnaufbau zu erleichtern. Zwischen Pfosten 16 und Folie 1 ist zusätzlich eine Beilagscheibe 18 angeordnet. Durch diese Beilagscheibe 18 kann ein Verschluss bzw. eine Abdichtung der Folie 1 in jenem Bereich der Folie 1 erzielt werden, durch den der Pfosten 16 durch die Folie 1 dringt (Durchdringungsbereich). Dies ist einerseits wichtig, um die Folie 1 gegenüber der Mundhöhle abzudichten und somit die Entstehung von Entzündungen zu hemmen. Andererseits kann damit auch erreicht werden, dass die Folie 1 in genau diesem sensiblen Durchdringungsbereich langsamer resorbiert und damit diesen Bereich besser schützen kann. Die Beilagscheibe 18 kann dabei aus Titan bestehen und das Implantat 14 radial überragen. Die Form der Beilagscheibe 18 kann beispielsweise rund oder oval sein. Die Beilagscheibe 18 kann auch zuschneidbar ausgebildet sein, um je nach Anwendungsfall einen optimalen Verschluss des Durchdringungsbereichs durch die Folie 1 herbeiführen zu können oder um den sensiblen defekten Bereich um das Implantat 14 zu stabilisieren. Dabei kann die Beilagscheibe 18 auch so hergestellt sein, dass die Folie 1 in einer Nut der Beilagscheibe 18 nach Bedarf eingeklemmt und verpresst werden kann, um eine Stabilisierung zu erreichen.

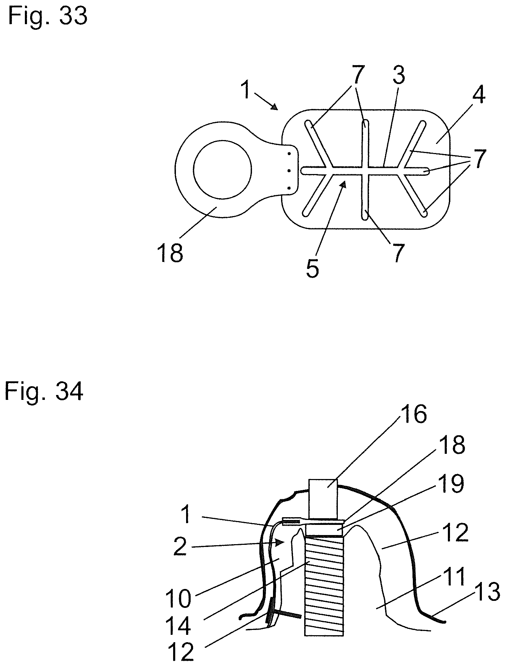

Figur 33 zeigt eine Folie 1, die in einer Nut einer Beilagscheibe 18 nach eingeklemmt und verpresst ist und Figur 34 zeigt die Anordnung dieser Folie 1 an einer Knochendefektstelle 2. Im gezeigten Beispiel überragt das obere Ende des Implantats 14 (Implantatkopf) den Kieferknochen 11 nicht, sondern befindet sich unterhalb des Knochenniveaus. Je nachdem, wie tief sich das obere Ende des Implantats 14 im Kieferknochen 11 befindet bzw. wie groß der Niveauunterschied zwischen Implantatkopf und Knochenniveau ist, kann durch verschieden hohe Einsätze 19 ein Ausgleich geschaffen werden, sodass die Folie 1 oder Beilagscheibe 18 ohne Kraterbildung fixiert werden kann.

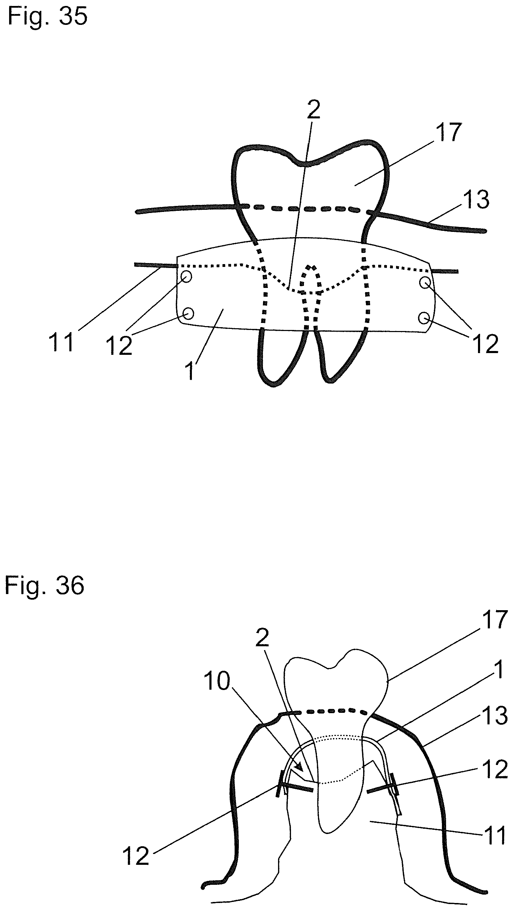

Figur 35 zeigt ein Beispiel einer Knochendefektstelle 2 um einen natürlichen Zahn 17, die mit einer vorgeschlagenen Folie 1 abgedeckt ist. In diesem Beispiel handelt es sich um die Anwendung der vorgeschlagenen Folie 1 zur Abdeckung einer parodontalen Konchendefektstelle 2. Die von der Folie 1 abgedeckten Abschnitte des Kieferknochens 11 und des Zahnes 17 sind dabei strichliert dargestellt.

Figur 36 zeigt eine Schnittdarstellung eines Kieferknochens 11 mit einer Knochendefektstelle 2 um einen natürlichen Zahn 17. Um einen Knochenaufbau und/oder einen Aufbau des Zahnhalteapparates an der Knochendefektstelle 2 zu ermöglichen, wird eine vorgeschlagene vorverbundene mehrschichtige Folie 1 über die Knochendefektstelle 2 und den Zahn 17 entsprechend angeformt und mittels entsprechender Befestigungsvorrichtungen 12 am Kieferknochen 11 verankert. Bei den Befestigungsvorrichtungen 12 kann es sich beispielsweise um bioresorbierbare Nägel handeln. Im sich bildenden Hohlraum 10 zwischen Folie 1 und Knochendefektstelle 2 bzw. Kieferknochen 11 können Knochenersatzmaterialien und/oder Träger für Medikamente, Wachstumsfaktoren und/oder sonstige die Heilung und Knochenbildung und Ausbildung eines Zahnhalteapparates um natürliche Zähne fördernde und schützende Substanzen enthalten sein, um die Knochenregeneration zu fördern. Nach Anbringung der Folie 1 wird das zuvor entfernte oder zurückgeklappte Zahnfleisch 13 wieder über die Folie 1 gedeckt und entsprechend vernäht. Aufgrund der Bioresorbierbarkeit der Folie 1 und der Befestigungsvorrichtungen 12 ist keine weitere Operation nötig, um die Folie 1 und/oder die Befestigungsvorrichtungen 12 nach erfolgtem Knochenaufbau wieder zu entfernen.

## Patentansprüche

1. Verfahren zur Herstellung einer vorverbundenen mehrschichtigen Folie (1), zur Abdeckung einer Knochendefektstelle (2), wobei die Folie (1) wenigstens eine im Wesentlichen vollständig bioresorbierbare Deckschicht (4, 4a, 4b) umfasst, wobei die wenigstens eine Deckschicht (4, 4a, 4b) an eine thermisch verformbare und im Wesentlichen vollständig bioresorbierbare Formschicht (3) angelegt wird, wobei die wenigstens eine Deckschicht (4, 4a, 4b) mit der Formschicht (3) thermisch und/oder mechanisch verbunden, vorzugsweise verpresst, wird, **dadurch gekennzeichnet, dass**
- an der Formschicht (3) dornenartige Vorsprünge (20) angeordnet sind, wobei durch das Anlegen der wenigstens einen Deckschicht (4, 4a, 4b) an die Formschicht (3) die Vorsprünge (20) in die wenigstens eine Deckschicht (4, 4a, 4b) gedrückt werden und/oder durch die wenigstens eine Deckschicht (4, 4a, 4b) hindurchgesteckt werden und/oder
- vor dem thermischen und/oder mechanischen Verbinden im Wesentlichen vollständig bioresorbierbare Verbindungsvorrichtungen (21), vorzugsweise Nieten oder Stifte, durch die Formschicht (3) und die wenigstens eine Deckschicht (4, 4a, 4b) hindurchgesteckt werden.

2. Verfahren nach Anspruch 1, **dadurch gekennzeichnet, dass** zur thermischen Verbindung der wenigstens einen Deckschicht (4, 4a, 4b) mit der Formschicht (3) zumindest die Formschicht (3) erwärmt wird, wobei vorzugsweise die Formschicht (3) auf eine Temperatur im Bereich von 50°C bis 70°C, vorzugsweise auf eine Temperatur von 60°C, erwärmt wird.

3. Verfahren nach Anspruch 1 oder 2, **dadurch gekennzeichnet, dass** die Vorsprünge (20) einstückig an die Formschicht (3) angeformt sind.

4. Verfahren nach einem der Ansprüche 1 bis 3, **dadurch gekennzeichnet, dass** die wenigstens eine Deckschicht (4, 4a, 4b) mit der Formschicht (3) verpresst wird, wobei vorzugsweise während des Verpressens wenigstens eine der Folie (1) zugewandte Fläche (23, 24) einer Pressvorrichtung (22) erwärmt wird, vorzugsweise auf eine Temperatur im Bereich von 50°C bis 70°C, besonders bevorzugt auf eine Temperatur von 60°C.

5. Vorverbundene mehrschichtige Folie (1) zur Abdeckung einer Knochendefektstelle (2), hergestellt nach einem Verfahren nach einem der Ansprüche 1 bis 4, wobei die Folie (1) wenigstens eine im Wesentlichen vollständig bioresorbierbare Deckschicht (4, 4a, 4b) umfasst, wobei die wenigstens eine Deckschicht (4, 4a, 4b) mit einer thermisch verformbaren und im Wesentlichen vollständig bioresorbierbaren Formschicht (3) thermisch und/oder mechanisch verbunden ist, **dadurch gekennzeichnet, dass**
- an der Formschicht (3) dornenartige Vorsprünge (20) angeordnet sind, wobei vorzugsweise die Vorsprünge (20) einstückig an die Formschicht (3) angeformt sind und/oder
- im Wesentlichen vollständig bioresorbierbare Verbindungsvorrichtungen (21), vorzugsweise Nieten oder Stifte, zur Verbindung der Formschicht (3) mit der wenigstens einen Deckschicht (4, 4a, 4b) durch die Formschicht (3) und die wenigstens eine Deckschicht (4, 4a, 4b) hindurchgesteckt sind.

6. Mehrschichtige Folie (1) nach Anspruch 5, **dadurch gekennzeichnet, dass** die wenigstens eine Deckschicht (4, 4a, 4b) zumindest teilweise, vorzugsweise im Wesentlichen vollständig, aus einem bioresorbierbaren Collagenmaterial besteht, wobei vorzugsweise das bioresorbierbare Collagenmaterial Typ-I-Collagen und/oder Typ-III-Collagen umfasst.

7. Mehrschichtige Folie (1) nach Anspruch 5 oder 6, **dadurch gekennzeichnet, dass** die Formschicht (3) zumindest teilweise, vorzugsweise im Wesentlichen vollständig, aus einem bioresorbierbaren Polymermaterial besteht.

8. Mehrschichtige Folie (1) nach Anspruch 7, **dadurch gekennzeichnet, dass** das bioresorbierbare Polymermaterial Milchsäure, vorzugsweise L-Milchsäure, und/oder deren Derivate umfasst, wobei vorzugsweise der Anteil der Milchsäure im bioresorbierbaren Polymermaterial mindestens 70%, vorzugsweise etwa 80% bis 95%, besonders bevorzugt im Wesentlichen etwa 82%, beträgt.

9. Mehrschichtige Folie (1) nach Anspruch 7 oder 8, **dadurch gekennzeichnet, dass** das bioresorbierbare Polymermaterial Glykolsäure umfasst, wobei vorzugsweise der Anteil der Glykolsäure im bioresorbierbaren Polymermaterial höchstens 30%, vorzugsweise etwa 15% bis 20%, besonders bevorzugt im Wesentlichen etwa 18%, beträgt.

10. Mehrschichtige Folie (1) nach einem der Ansprüche 5 bis 9, **dadurch gekennzeichnet, dass** die Formschicht (3) und die wenigstens eine Deckschicht (4, 4a, 4b) unterschiedliche Flächenausdehnungen aufweisen, wobei vorzugsweise die Formschicht (3) eine geringere Flächenausdehnung als die wenigstens eine Deckschicht (4, 4a, 4b) einnimmt.

11. Mehrschichtige Folie (1) nach einem der Ansprüche 5 bis 10, **dadurch gekennzeichnet, dass** die wenigstens eine Deckschicht (4, 4a, 4b) und/oder die Formschicht (3) durchgehend flächig ausgebildet ist bzw. sind.

12. Mehrschichtige Folie (1) nach einem der Ansprüche 5 bis 11, **dadurch gekennzeichnet, dass** die Formschicht (3) zur Anformung an die Knochendefektstelle (2) eine Formstruktur (5) aufweist, wobei vorzugsweise die Formstruktur (5) wenigstens abschnittsweise einen konvex und/oder konkav gekrümmten Rand (6) und/oder wenigstens abschnittsweise eine konvex und/oder konkav gekrümmte Form aufweist.

13. Mehrschichtige Folie (1) nach Anspruch 12, **dadurch gekennzeichnet, dass** die Formstruktur (5) wenigstens ein strebenförmiges Anformelement (7) aufweist.

14. Mehrschichtige Folie (1) nach Anspruch 12 oder 13, **dadurch gekennzeichnet, dass** die Formstruktur (5) im Wesentlichen gitterförmig ausgebildet ist.

15. Mehrschichtige Folie (1) nach einem der Ansprüche 12 bis 14, **dadurch gekennzeichnet, dass** die Formstruktur (5) durch wenigstens eine Verstärkung der Formschicht (3) ausgebildet ist.

16. Mehrschichtige Folie nach einem der Ansprüche 5 bis 15, **dadurch gekennzeichnet, dass** die Folie (1) zusätzlich eine Trägerschicht (8) für wenigstens eine darauf anzuordnende oder angeordnete Substanz - vorzugsweise Medikamente, Wachstumsfaktoren und/oder sonstige die Heilung und Knochenbildung fördernde und schützende Substanzen - aufweist.

## Claims

1. A method of producing a pre-bonded multilayer film (1) for covering a bone defect site (2), wherein the film (1) includes at least one substantially completely bioresorbable cover layer (4, 4a, 4b), wherein the at least one cover layer (4, 4a, 4b) is applied to a thermally deformable and substantially completely bioresorbable molding layer (3), wherein the at least one cover layer (4, 4a, 4b) is thermally and/or mechanically connected, preferably pressed, to the molding layer (3), **characterised in that**
- arranged on the molding layer (3) are spike-like projections (20), wherein by applying the at least one cover layer (4, 4a, 4b) to the molding layer (3) the projections (20) are pressed into the at least one cover layer (4, 4a, 4b) and/or passed through the at least one cover layer (4, 4a, 4b), and/or
- prior to the thermal and/or mechanical connection being made substantially completely bioresorbable connecting devices (21), preferably rivets or pins, are passed through the molding layer (3) and the at least one cover layer (4, 4a, 4b).

2. A method as set forth in claim 1 **characterised in that** at least the molding layer (3) is heated for thermally connecting the at least one cover layer (4, 4a, 4b) to the molding layer (3), wherein preferably the molding layer (3) is heated to a temperature in the range of 50°C to 70°C, preferably to a temperature of 60°C.

3. A method as set forth in claim 1 or 2 **characterised in that** the projections (20) are formed in one piece on the molding layer (3).

4. A method as set forth in one of claims 1 through 3 **characterised in that** the at least one cover layer (4, 4a, 4b) is pressed to the molding layer (3), wherein preferably during the pressing operation at least one surface (23, 24) of a pressing apparatus (22), that is towards the film (1), is heated, preferably to a temperature in the range of 50°C to 70°C, particularly preferably to a temperature of 60°C.

5. A pre-bonded multilayer film (1) for covering a bone defect site (2), produced by a method as set forth in one of claims 1 through 4, wherein the film (1) includes at least one substantially completely bioresorbable cover layer (4, 4a, 4b), wherein the at least one cover layer (4, 4a, 4b) is thermally and/or mechanically connected to a thermally deformable and substantially completely bioresorbable molding layer (3), **characterised in that**
- arranged on the molding layer (3) are spike-like projections (20), wherein preferably the projections (20) are formed in one piece on the molding layer (3), and/or
- substantially completely bioresorbable connecting devices (21), preferably rivets or pins, are passed through the molding layer (3) and the at least one cover layer (4, 4a, 4b) for connecting the molding layer (3) with the at least one cover layer (4, 4a, 4b).

6. A multilayer film (1) as set forth in claim 5 **characterised in that** the at least one cover layer (4, 4a, 4b) at least partially and preferably substantially completely comprises a bioresorbable collagen material, wherein preferably the bioresorbable collagen material includes type-I-collagen and/or type-III-collagen.

7. A multilayer film (1) as set forth in claim 5 or 6 **characterised in that** the molding layer (3) at least partially and preferably substantially completely comprises a bioresorbable polymer material.

8. A multilayer film (1) as set forth in claim 7 **characterised in that** the bioresorbable polymer material includes lactic acid, preferably L-lactic acid, and/or derivatives thereof, wherein preferably the proportion of lactic acid in the bioresorbable polymer material is at least 70%, preferably about 80% to 95%, particularly preferably substantially about 82%.

9. A multilayer film (1) as set forth in claim 7 or 8 **characterised in that** the bioresorbable polymer material includes glycolic acid, wherein preferably the proportion of glycolic acid in the bioresorbable polymer material is at most 30%, preferably about 15% to 20%, particularly preferably substantially about 18%.

10. A multilayer film (1) as set forth in one of claims 5 through 9 **characterised in that** the molding layer (3) and the at least one cover layer (4, 4a, 4b) are of different surface areas, wherein preferably the molding layer (3) occupies a smaller surface area than the at least one cover layer (4, 4a, 4b).

11. A multilayer film (1) as set forth in one of claims 5 through 10 **characterised in that** the at least one cover layer (4, 4a, 4b) and/or the molding layer (3) is or are laminar throughout.

12. A multilayer film (1) as set forth in one of claims 5 through 11 **characterised in that** the molding layer (3) has a shaping structure (5) for shaping molding to the bone defect site (2), wherein preferably the shaping structure (5) has at least portion-wise a convexly and/or concavely curved edge (6) and/or at least portion-wise a convexly and/or concavely curved shape.

13. A multilayer film (1) as set forth in claim 12 **characterised in that** the shaping structure (5) has at least one strut-shaped shaping molding element (7).

14. A multilayer film (1) as set forth in claim 12 or 13 **characterised in that** the shaping structure (5) is substantially grid-shaped.

15. A multilayer film (1) as set forth in one of claims 12 through 14 **characterised in that** the shaping structure (5) is provided by at least one reinforcement of the molding layer (3).

16. A multilayer film as set forth in one of claims 5 through 15 **characterised in that** the film (1) additionally has a carrier layer (8) for at least one substance which is arranged or which is to be arranged thereon - preferably drugs, growth factors and/or other substances for promoting and protecting healing and bone formation.

## Revendications

1. Procédé de fabrication d'un film multicouche pré-relié (1) pour le recouvrement d'une zone de défaut osseux (2), dans lequel le film (1) comprend au moins une couche de recouvrement (4, 4a, 4b) essentiellement biorésorbable intégralement, dans lequel la au moins une couche de recouvrement (4, 4a, 4b) est appuyée sur une couche moulée (3) déformable thermiquement et essentiellement biorésorbable intégralement, dans lequel la au moins une couche de recouvrement (4, 4a, 4b) est reliée, de préférence pressée, thermiquement et/ou mécaniquement à la couche moulée (3), **caractérisé en ce que**
- sur la couche moulée (3) sont disposées des saillies en forme d'épine (20), dans lequel par l'appui de la au moins une couche de recouvrement (4, 4a, 4b) sur la couche moulée (3), les saillies (20) sont poussées dans la au moins une couche de recouvrement (4, 4a, 4b) et/ou sont passées à travers la au moins une couche de recouvrement (4, 4a, 4b) et/ou,
- avant la liaison thermique et/ou mécanique, des dispositifs de liaison (21) essentiellement biorésorbables intégralement, de préférence, des rivets ou des broches, sont passés à travers la couche moulée (3) et la au moins une couche de recouvrement (4, 4a, 4b).

2. Procédé selon la revendication 1, **caractérisé en ce que** pour la liaison thermique de la au moins une couche de recouvrement (4, 4a, 4b) avec la couche moulée (3) au moins la couche moulée (3) est chauffée, dans lequel, de préférence, la couche moulée (3) est chauffée à une température allant de 50°C à 70°C, de préférence à une température de 60°C.

3. Procédé selon la revendication 1 ou 2, **caractérisé en ce que** les saillies (20) sont formées d'une seule pièce sur la couche moulée (3).

4. Procédé selon l'une des revendications 1 à 3, **caractérisé en ce que** la au moins une couche de recouvrement (4, 4a, 4b) est pressée avec la couche moulée (3), dans lequel de préférence, pendant le pressage, au moins une surface (23, 24), orientée vers le film (1), d'un dispositif de pressage (22) est chauffée, de préférence, à une température allant de 50°C à 70°C, de préférence encore à une température de 60°C.

5. Film multicouche pré-relié (1) pour le recouvrement d'une zone de défaut osseux (2), fabriquée selon un procédé selon l'une des revendications 1 à 4, dans lequel le film (1) comprend au moins une couche de recouvrement (4, 4a, 4b) essentiellement biorésorbable intégralement, dans lequel la au moins une couche de recouvrement (4, 4a, 4b) est reliée thermiquement et/ou mécaniquement à une couche moulée (3) déformable thermiquement et essentiellement biorésorbable intégralement, **caractérisé en ce que**
- sur la couche moulée (3) sont disposées des saillies en forme d'épine (20), dans laquelle, de préférence, les saillies (20) sont formées d'une seule pièce sur la couche moulée (3) et/ou
- des dispositifs de liaison (21) essentiellement biorésorbables intégralement, de préférence, des rivets ou des broches, pour lier la couche moulée (3) à la au moins une couche de recouvrement (4, 4a, 4b) sont passés à travers la couche moulée (3) et la au moins une couche de recouvrement (4, 4a, 4b).

6. Film multicouche (1) selon la revendication 5, **caractérisé en ce que** la au moins une couche de recouvrement (4, 4a, 4b) est composée au moins partiellement, de préférence essentiellement intégralement, d'un matériau collagénique biorésorbable, dans lequel, de préférence, le matériau collagénique biorésorbable comprend du collagène de type I et/ou du collagène de type III.

7. Film multicouche (1) selon la revendication 5 ou 6, **caractérisé en ce que** la couche moulée (3) est composée au moins partiellement, de préférence essentiellement intégralement, d'un matériau polymère biorésorbable.

8. Film multicouche (1) selon la revendication 7, **caractérisé en ce que** le matériau polymère biorésorbable comprend des acides lactiques, de préférence, des acides lactiques L, et/ou des dérivés de ceux-ci, dans lequel, de préférence, la part de l'acide lactique dans le matériau polymère biorésorbable s'élève au moins à 70 %, de préférence à 80 % jusqu'à 95 % environ, de préférence encore essentiellement à 82 %.

9. Film multicouche (1) selon la revendication 7 ou 8, **caractérisé en ce que** le matériau polymère biorésorbable comprend de l'acide glyoxylique, dans lequel, de préférence, la part de l'acide glyoxylique dans le matériau polymère biorésorbable est de 30 % au maximum, de préférence de 15 à 20 % environ, de préférence encore essentiellement de 18 % environ.

10. Film multicouche (1) selon l'une des revendications 5 à 9, **caractérisé en ce que** la couche moulée (3) et la au moins une couche de recouvrement (4, 4a, 4b) présentent des superficies différentes, dans lequel, de préférence, la couche moulée (3) occupe une superficie moindre que la au moins une couche de recouvrement (4, 4a, 4b).

11. Film multicouche (1) selon l'une des revendications 5 à 10, **caractérisé en ce que** la au moins une couche de recouvrement (4, 4a, 4b) et/ou la couche moulée (3) est ou sont conçue(s) planes en continu.

12. Film multicouche (1) selon l'une des revendications 5 à 11, **caractérisé en ce que** la couche moulée (3) pour la mise en forme sur la zone de défaut osseux (2) présente une structure moulée (5), dans lequel, de préférence, la structure moulée (5) présente au moins par endroits un bord recourbé de façon convexe et/ou concave (6) et/ou au moins, par endroits, une forme recourbée de façon convexe et/ou concave.

13. Film multicouche (1) selon la revendication 12, **caractérisé en ce que** la structure moulée (5) présente au moins un élément de formage en forme d'entretoise (7).

14. Film multicouche (1) selon la revendication 12 ou 13, **caractérisé en ce que** la structure moulée (5) est conçue essentiellement en forme de grille.

15. Film multicouche (1) selon l'une des revendications 12 à 14, **caractérisé en ce que** la structure moulée (5) est conçue par au moins un renforcement de la couche moulée (3).

16. Film multicouche selon l'une des revendications 5 à 15, **caractérisé en ce que** le film (1) présente, en outre, une couche support (8) pour au moins une substance à disposer ou disposée dessus, de préférence des médicaments, des facteurs de croissance et/ou d'autres substances stimulant et protégeant la cicatrisation et la formation osseuse.
